(19) Europäisches Patentamt
European Patent Office
Office européen des brevets

(11) Publication number: 0 230 916 B1

(12) **EUROPEAN PATENT SPECIFICATION**

(45) Date of publication of patent specification: **21.08.91 Bulletin 91/34**

(51) Int. Cl.[5]: **C07C 69/675**, C07C 67/31

(21) Application number: **87100457.8**

(22) Date of filing: **15.01.87**

(54) **Process for the preparation of the esters of an alkyltartronic acid.**

(30) Priority: **16.01.86 IT 1909786**

(43) Date of publication of application: **05.08.87 Bulletin 87/32**

(45) Publication of the grant of the patent: **21.08.91 Bulletin 91/34**

(84) Designated Contracting States: **BE CH DE FR GB LI NL**

(56) References cited:
**EP-A- 0 166 348**
**DE-A- 2 524 222**

(73) Proprietor: **ISTITUTO GUIDO DONEGANI S.p.A.**
**Via Caduti del Lavoro**
**I-28100 Novara (IT)**

Proprietor: **CONSIGLIO NAZIONALE DELLE RICERCHE**
**Piazzale Aldo Moro, 7**
**I-00198 Roma (IT)**

(72) Inventor: **Santi, Roberto**
**24 F, V.le P.zza D'Armi**
**I-28100 Novara (IT)**
Inventor: **Cometti, Giuseppe**
**14, Via Crocetta**
**I-28048 Verbania Pallanza Novara (IT)**
Inventor: **Pagani, Anselmo**
**21, P.zza Cattaneo**
**I-28069 Trecate Novara (IT)**

(74) Representative: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40 (DE)**

EP 0 230 916 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

It is known to prepare the esters of alkyltartronic acids having formula (I) :

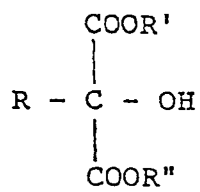

(I)

wherein R is an alkyl or cycloalkyl group and wherein R′ and R″, the same or different from each other, are organic groups inert towards the reaction medium, by oxidation of the corresponding alkyl-malonic esters having formula (II) :

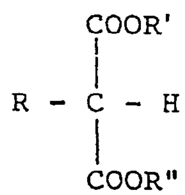

(II)

using, as an oxidizing agent, 98% $HNO_3$ (see US-A-3,997,593). It is also known to alkylate mesoxalic esters with a Cd-dialkyl compound or with trialkylphosphites (Arbuzov reaction).

According to other methods such esters are prepared by hydrolysis of meta-bromo-malonic acid with baryta and subsequent esterification with an alcohol, or by acidic alcoholysis of alpha-cyanolactic acid (pyruvic cyanohydrin) or of 2-acetoxy-2-methyl-malononitrile (1-acetoxy-1, 1-dicyano-ethane).

Finally, according to a recent method which is described in EP-A-166 348, said esters are prepared by hydroxylating methacrylic acid to alpha-methyl-glyceric acid with $H_2O_2$ and in the presence of $H_2WO_4$, and by subsequent oxidation to methyl-tartronic acid, using $HNO_3$ or $O_2$ in the presence of either Pd or Pt, followed by an esterification with a suitable alcohol.

It has now been found that it is possible to obtain the alkyl-tartronic esters of formula (I) by direct oxidation of the alkyl-malonic esters of formula (II) without any toxic or dangerous oxidizing agent such as, e.g., $HNO_3$, and without any use of noble metals, using extremely simple procedures.

In its broadest aspect, the present invention provides a process for the preparation of esters of alkyl-tartronic acids having formula (I) by direct oxidation of the corresponding alkyl-malonic esters of formula (II) which is characterized in that the oxidation is carried out at 30-200°C with oxygen, air or other $O_2$-containing gases, in the presence of a catalytic system comprising :

a) an inorganic salt (preferably selected from halides, carbonates and bicarbonates) or an organic salt of a transition metal selected from manganese, iron, cobalt and copper ;

b) an alkali-metal salt of an aliphatic carboxylic acid, in amounts up to 60 moles per mole of transition metal ; optionally in the presence of a polar solvent having a dipolar moment equal to or higher than 1.68 Debye units.

The groups R′ and R″ of formula (I) and (II) can be, e.g. alkyl groups containing from 1 to 6 carbon atoms, cycloalkyl, aryl, arylalkyl or alkylaryl groups.

The reaction can be performed under atmospheric pressure, with an oxygen (or air) flow rate of about 2 $m^3/h$ per $m^3$ of reaction volume, or under a pressure ranging from 1 to 50 bar.

Salt (a) preferably is selected from the salts of aliphatic mono-carboxylic acids, preferably containing 1 to 22 carbon atoms, and among these the acetates are particularly preferred.

Particularly advantageous is the use of a cobalt salt in combination with a manganese salt in a molar ratio from 1 :9 to 9 :1 (expressed in terms of metal). Generally, amounts from 0.03 to 0.003 moles of transition metal per mole of alkyl-malonic ester can be used. Preferably, the alkali-metal salt (b) is selected from sodium and potassium acetates and preferably is used in amounts from 10 to 20 moles per mole of transition metal.

The polar solvent, in which also the catalyst should be soluble, preferably is selected from aliphatic carboxylic acids containing up to 5 carbon atoms, optionally in admixture with water. Best results were obtained by using acetic acid.

The reaction temperature should generally be from 30° to 200°C, depending on the composition of the reaction mixture and on the predetermined reaction rate. The addition of up to 50% by weight, preferably at least 10% by weight, of an alcohol having from 2 to 6 carbon atoms (e.g. tert.-butanol) satisfactorily inhibits the cleavage of the alkyl-malonic ester used as the starting material, thus promoting the recovery of the unreacted material. At the end of the reaction, the alkyl-tartronic ester can be stripped from the reaction mixture by substantially usual techniques such as, e.g., distillation. It is also possible to recover the catalyst from the reaction mixture and to recycle said catalyst to the oxidation reactor. The process according to the invention, contrary to the processes used until now, allows to obtain alkyl-tartronic esters of formula (I) with good yields, avoiding the use of noble metals and of either toxic or dangerous oxidizing agents, with a good control of the course of the reaction, and in a very simple manner which is very suitable for exploitation on an industrial scale.

The esters having formula (I) are particularly useful when R is $CH_3$ and when R′ = R″ are alkyl groups having from 1 to 4 carbon atoms. In this case they can be used, in fact, as intermediates for the preparation of amino acids, of barbituric derivatives and of oxazolidinyl derivatives having a pesticidal or analgesic effect. In particular, the diethyl ester of methyltartronic acid is an intermediate for the preparation of a fungicide known under the trade name "SERINAL"®.

Some Examples are now given for illustrative purposes, but are by no way limitative of the scope of the present invention.

## EXAMPLE 1

A glass reactor equipped with stirrer, reflux condenser and temperature control system was charged with

0.125 g (0.7 mmol) of $Co(CH_3COO)_2 \cdot 4H_2O$;

0.125 g (0.7 mmol) of $Mn(CH_3COO)_2 \cdot 4H_2O$;

0.75 g (9 mmol) of $NaOCOCH_3$;

5 g (28.7 mmol) of diethyl methylmalonate dissolved in 5 ml of acetic acid, 1 ml of water and 1 ml of tertiary butyl alcohol.

Through the mixture 7 $dm^3/h$ of oxygen were bubbled while keeping the reaction mixture at 130°C by means of an external oil bath. Five hours later, at the end of the reaction, the solvents were removed under vacuum and the residue was distilled under reduced pressure. A fraction was obtained which contained 2.4 g of diethyl methylmalonate and 2.4 g of diethyl methyltartronate, corresponding to a 52% conversion and a 84% selectivity to diethyl methyltartronate and thus a yield of 43.9%.

## EXAMPLE 2

The reactor of Example 1 was charged with :

0.125 g (0.7 mmol) of $Co(CH_3COO)_2.4H_2O$;

0.125 g (0.7 mmol) of $Mn(CH_3COO)_2.4H_2O$;

0.75 g (9 mmol) of $NaOCOCH_3$;

5 g (28.7 mmol) of diethyl methylmalonate;

10 ml of acetic acid; and

1 ml of water.

Then the procedure of Example 1 was followed. After the destillation there were obtained :

2.45 g of diethyl methyltartronate ;
0.72 g of diethyl methylmalonate ; and
0.4 g of residue ;
corresponding to 85.6% conversion, a 52.4% selectivity and a 45% yield.

EXAMPLE 3

Repeating the procedure of Examples 1 and 2, the reactor was charged with :

0.125 g (0.7 mmol) of $Mn(CH_3COO)_2.4H_2O$;

0.125 g (0.7 mmol) of $Co(CH_3COO)_2.4H_2O$;

0.75 g (9 mmol) of $NaOCOCH_3$;

5 g (28.7 mmol) of diethyl methylmalonate; and

10 ml of acetic acid.

After the distillation there were obtained :

2.7 g of diethyl methyltartronate ;
0.27 g of diethyl methylmalonate ; and
0.7 g of residue ;
corresponding to a 94.6% conversion, a 52.3% selectivity and a 49.5% yield.

**Claims**

1. A process for the preparation of esters of alkyltartronic acids having formula (I) :

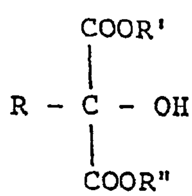

(I)

wherein R is an alkyl or cycloalkyl group and wherein R′ and R", the same or different from each other, are

organic groups inert towards the reaction medium ;
by direct oxidation of the corresponding alkyl-malonic esters having formula (II) :

```
      COOR'
      |
R  -  C  -  H          (II)
      |
      COOR"
```

characterized in that the oxidation is carried out at 30 to 200°C with oxygen, air or other $O_2$-containing gases in the presence of a catalytic system comprising :

a) an inorganic or organic salt of a transition metal selected from manganese, iron, cobalt and copper ;

b) an alkali-metal salt of an aliphatic carboxylic acid, in amounts up to 60 moles per mole of transition metal;

optionally in the presence of a polar solvent having a dipolar moment equal to or higher than 1.68 Debye units.

2. A process according to claim 1, wherein also an aliphatic alcohol having from 2 to 6 carbon atoms is present in amounts of up to 50% by weight with respect to the alkyl-malonic ester to be oxidized.

3. A process according to one or both of claims 1 and 2, wherein the substituents R′ and R″ are selected from alkyl groups containing from 1 to 6 carbon atoms, cycloalkyl, aryl, arylalkyl and alkylaryl groups.

4. A process according to one or more of claims 1 to 3, wherein the salts of said transition metals are salts of aliphatic mono-carboxylic acids, e.g. selected from Mn, Fe, Co and Cu acetates.

5. A process according to one or more of claims 1 to 4, wherein a cobalt salt is used in combination with a manganese salt in a molar ratio, expressed in terms of metal, from 1 :9 to 9 :1.

6. A process according to one or more of claims 1 to 5, wherein an amount from 0.03 to 0.003 moles of transition metal per mole of alkyl-malonic ester is used.

7. A process according to one or more of claims 1 to 6, wherein from 10 to 20 moles of alkali-metal salt per mole of transition metal are used.

8. A process according to one or more of claims 1 to 7, wherein the alkali-metal salt is selected from sodium acetate and potassium acetate.

9. A process according to one or more of claims 1 to 8, wherein the solvent is selected from aliphatic carboxylic acids containing up to 5 carbon atoms, e.g., acetic acid, optionally in admixture with $H_2O$.

10. A process according to one or more of claims 1 to 9, wherein an aliphatic alcohol containing from 2 to 6 carbon atoms, e.g., tert.-butanol, is added to the reaction mixture.

## Patentansprüche

1. Verfahren zur Herstellung von Estern der Alkyltartronsäuren der Formel (I)

```
      COOR'
      |
R  -  C  -  OH          (I)
      |
      COOR"
```

worin R eine Alkyl- oder Cycloalkylgruppe ist und R′ und R″, die gleich oder voneinander verschieden sein können, organische, gegenüber dem Reaktionsmedium inerte Gruppen sind,
durch direkte Oxidation der entsprechenden Alkylmalonester der Formel (II) :

```
      COOR'
      |
R  -  C  -  H          (II)
      |
      COOR"
```

dadurch gekennzeichnet, daß die Oxidation bei 30 bis 200°C mit Sauerstoff, Luft oder anderen $O_2$ enthaltenden

Gasen in Anwesenheit eines Katalysatorsystems, das

a) ein anorganisches oder organisches Salz eines Übergangsmetalls, ausgewählt aus Mangan, Eisen, Kobalt und Kupfer,

b) ein Alkalimetallsalz einer aliphatischen Carbonsäure in Mengen bis zu 60 Mol pro Mol Übergangsmetall umfaßt, und wahlweise in Anwesenheit eines polaren Lösungsmittels mit einem Dipol-Moment gleich oder höher als 1,68 Debye-Einheiten durchgeführt wird.

2. Verfahren nach Anspruch 1, worin ferner ein aliphatischer Alkohol mit 2 bis 6 Kohlenstoffatomen in Mengen bis zu 50 Gew.%, bezogen auf den zu oxidierenden Alkylmalonester, anwesend ist.

3. Verfahren nach einem oder beiden der Ansprüche 1 und 2, worin die Substituenten R′ und R″ aus Alkylgruppen mit 1 bis 6 Kohlenstoffatomen, Cycloalkyl-, Aryl-, Arylalkyl- und Alkylarylgruppen ausgewählt werden.

4. Verfahren nach einem oder mehreren der Ansprüche 1 bis 3, worin die Salze der Übergangsmetalle Salze von aliphatischen Monocarbonsäuren, z.B. ausgewählt aus Mn-, Fe-, Co- und Cu-Acetaten, sind.

5. Verfahren nach einen oder mehreren der Ansprüche 1 bis 4, worin ein Kobaltsalz in Kombination mit einem Mangansalz in einem molaren Verhältnis, bezogen auf das Metall, von 1 :9 bis 9 :1 verwendet wird.

6. Verfahren nach einem oder mehreren der Ansprüche 1 bis 5, worin eine Menge von 0,03 bis 0,003 Mol Übergangsmetall pro Mol Alkylmalonester verwendet wird.

7. Verfahren nach einem oder mehreren der Ansprüche 1 bis 6, worin 10 bis 20 Mol Alkalimetallsalz pro Mol Übergangsmetall verwendet werden.

8. Verfahren nach einem oder mehreren der Ansprüche 1 bis 7, worin das Alkalimetallsalz aus Natriumacetat und Kaliumacetat ausgewählt wird.

9. Verfahren nach einem oder mehreren der Ansprüche 1 bis 8, worin das Lösungsmittel aus aliphatischen Carbonsäuren mit bis zu 5 Kohlenstoffatomen, z.B. Essigsäure, wahlweise in Mischung mit $H_2O$, ausgewählt wird.

10. Verfahren nach einem oder mehreren der Ansprüche 1 bis 9, worin ein aliphatischer Alkohol mit 2 bis 6 Kohlenstoffatomen, z.B. t-Butanol, der Reaktionsmischung zugefügt wird.

## Revendications

1. Un procédé pour la préparation d'esters d'acides alkyltartroniques représentés par la formule (I) :

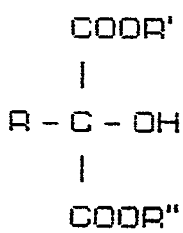

(I)

dans laquelle R est un groupe alkyle ou cycloalkyle et R′ et R″ qui peuvent être identiques ou différents, sont des groupes organiques inertes vis-à-vis du milieu réactionnel,
par oxydation directe des esters alkyl-maloniques correspondants de formule (II) :

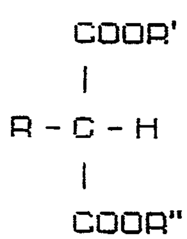

(II)

caractérisé en ce que l'oxydation est effectué entre 30° et 200°C avec de l'oxygène, de l'air ou d'autres gaz contenant de l'oxygène, en présence d'un système catalytique comprenant :

a) un sel inorganique ou organique d'un metal de transition choisi parmi le manganèse, le fer, le cobalt et le cuivre ;

b) un sel de métal alcalin d'un acide carboxylique aliphatique, en des quantités allant jusqu'à 60 moles par mole de métal de transition ;

éventuellement en présence d'un solvant polaire ayant un moment dipolaire égal ou supérieur à 1,68 unités Debye.

2. Un procédé suivant la revendication 1, caractérisé en ce qu'un alcool aliphatique ayant de 2 à 6 atomes de carbone est aussi présent en des quantités allant jusqu'à 50% en poids par rapport à l'ester alkyl-malonique à oxyder.

3. Un procédé suivant les revendications 1 et/ou 2, caractérisé en ce que les substituants R' et R" sont choisis parmi des groupes alkyles contenant 1 à 6 atomes de carbone, cycloalkyles, aryles, arylalkyles et alkylaryles.

4. Un procédé suivant l'une quelconque des revendications 1 à 3, caractérisé en ce que les sels des dits métaux de transition sont des sels d'acides mono-carboxyliques aliphatiques, choisis parmi les acétates de Mn, Fe, Co et Cu.

5. Un procédé suivant l'une quelconque des revendications 1 à 4, caractérisé en ce qu'un sel de cobalt est utilisé en combinaison avec un sel de manganèse selon un rapport molaire de 1/9 à 9/1, exprimé en termes de métaux.

6. Un procédé suivant l'une quelconque des revendications 1 à 5, caractérisé en ce qu'on utilise une quantité de 0,03 à 0,003 mole de métal de transition par mole d'ester alkyl-malonique.

7. Un procédé suivant l'une quelconque des revendications 1 à 6, caractérisé en ce qu'on utilise de 10 à 20 moles de sel de métal alcalin par mole de métal de transition.

8. Un procédé suivant l'une quelconque des revendications 1 à 7, caractérisé en ce que le sel de métal alcalin est choisi parmi l'acétate de sodium et l'acétate de potassium.

9. Un procédé suivant l'une quelconque des revendications 1 à 8, caractérisé en ce que le solvant est choisi parmi des acides carboxyliques aliphatiques contenant jusqu'à 5 atomes de carbone, comme l'acide acétique, éventuellement en mélange avec de l'eau.

10. Un procédé suivant l'une quelconque des revendications 1 à 9, caractérisé en ce qu'on ajoute au mélange réactionnel un alcool aliphatique contenant 2 à 6 atomes de carbone, comme le t-butanol.